# EUROPEAN PATENT SPECIFICATION

(11) **EP 1 125 561 B1**
(45) Date of publication and mention of the grant of the patent: **01.04.2009**
(21) Application number: 01301337.0
(22) Date of filing: 15.02.2001
(51) Int. Cl.: A61F 2/30, A61F 2/36

(54) **Metal jacket for a cementless artificial joint stem and artificial joint having the metal jacket**
Metallhülse für einen zementfreien künstlichen Gelenkschaft und künstliches Gelenk mit der Metallhülse
Manchon métallique pour une tige articulaire artificielle non cimentée et articulation artificielle avec le manchon métallique

(30) Priority: 16.02.2000 KR 2000007363
(43) Date of publication of application: 22.08.2001
(73) Proprietor: Korea Advanced Institute of Science and Technology, Taejon 305-701 (KR)
(72) Inventor: Yoon, Yong-San, Yusong-gu, Taejon 305-555 (KR)
(74) Representative: Moore, Christopher Mark

(56) References cited:
- EP-A- 0 016 480
- EP-A- 0 179 626
- EP-A- 0 278 807
- WO-A-96/29958
- DE-A- 2 842 847
- DE-A- 19 829 589
- DE-U- 29 501 042
- FR-A- 2 199 967
- FR-A- 2 215 927
- FR-A- 2 519 248
- GB-A- 2 024 631
- US-A- 4 064 567
- US-A- 4 728 335

## Description

The present invention relates to cementless artificial joint stems, particularly to a metal jacket for a cementless artificial joint stem, and to a cementless artificial joint having the artificial joint stem enclosed in such a jacket.

Generally, an artificial hip joint, for example, consists of an acetabular part and a femoral or thigh bone part, wherein the acetabular and femoral parts are made from either metal, plastics, or ceramic, independently.

The human femur is formed of soft cancellous bone in the metaphyseal region and hard cortical bone in the diaphyseal region. Therefore, in order to insert an artificial joint into the human body, an operation must be conducted to ream out the bone canal at the proximal site, the stem of artificial joint being inserted into the reamed-out site and finally anchored by using cement or, when cement is not used, a stem having a porous or roughened surface layer on an outer circumference thereof is inserted so as to allow that surface to become physiologically interlocked with adjacent bone as it grows.

Of the two methods described above, the method in which an artificial joint is secured using cement is disclosed in Korean patent publication No. 8501814 entitled "Torsion resistant artificial hip joint".

WO 96/29958 describes an endoprosthesis for an artificial joint comprising an artificial endoprosthesis shaft and at least one functional element formed at the end of the shaft. The shaft is guided inside an implantable sheath so as to be capable of moving.

FR 2199 967 describes a bone prosthesis having a keying electrode, which electrode has key formations into which bone may grow for locking engagement therewith.

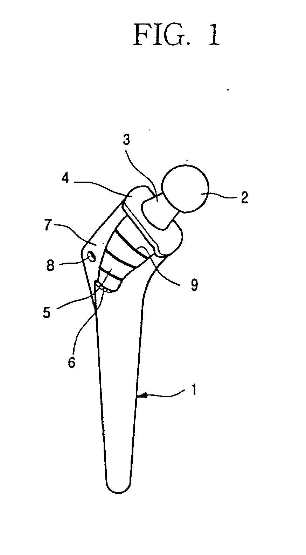

Referring to Figure 1, the art disclosed in our prior patent is briefly reviewed.

The artificial joint 1 is integrally formed, and comprises a head 2, neck 3, collar 4 and stem 5. The leading end of the collar 4 is curved to be secured tightly within the inner top edge of the cortex of the femur. The stem 5 comprises a curved column with a cross-section at the top, as viewed, resembling an ellipse which gradually changes to a circle at the bottom, as viewed. The shape of the stem 5 is adapted to prevent rotation thereof under compressive forces acting vertically from the top of joint and lateral forces acting perpendicularly with respect to the stem 5 of the joint 1 at the head 2.

Further, on the upper external surface of the stem 5 a blade 7 with an appropriate thickness is provided. The blade 7 protrudes longitudinally to prevent the joint 1 from turning in the femur, even in circumstances where a torque is generated in an arbitrary direction after surgery. A fixing hole 8 is formed in the centre of the blade 7 to aid retention thereof.

On the inward side of the blade 7, the stem 5 is formed with a number of longitudinally spaced apart lateral grooves. Iron wires 9, in chain form, are inserted into the grooves so as to wrap the stem 5. Cement 6 is coated, to a predetermined thickness, onto the surface of the stem 5, including the chain-like wires 9. This pre-coating the stem 5 and wires 9 with cement 6 facilitates adhesion with cement used in the surgical operation and reduces the heat generated during the curing period.

In order to introduce such an artificial joint into a human long bone, reaming is carried out at the bone canal beforehand so that the artificial joint 1 can be inserted after an appropriate amount of cement is injected into the bone canal. The stem 5 is inserted into the cement-injected site, so that this cement may adhere with the pre-coated cement layer 6 on the surface of the stem 5, with the result that the stem 5 is firmly secured in the long bone such as the femur.

An artificial joint 1 using cement as described is weak due to the fragile inter-cement connection between that on the joint stem 5 and that injected into the bone canal. To reduce this drawback, the connection between the femur and the joint stem was modified so as to be physiologically interlocked at the joint stem with the in-growth or on-growth of bone into and/or on to a porous or roughened surface of the stem. In this technique a joint stem, the surface of which is either made porous or roughened through grit blasting with fine media, is inserted surgically into the bone canal. With gradual growth of the bone, the bone becomes physiologically interlocked with the porous or roughened surface layer, to fix the stem in the bone, eg. the femur. Such a cementless artificial joint, is better than an artificial joint using cement, because it provides a strong interface in a biologically active young patient. However, this process of growth of bone in to or on to the joint stem may be impeded due to there being a gap, to the order of several millimetres, between the stem and walls of the bone canal.

In addition, the vertical force acting on the head of the stem may encourage shear forces at the interface of the bone and stem such that a fibrous tissue membrane develops on the bone, thereby forming an effective joint space. This can allow the wear particles, produced at the plastic cup, to penetrate into the gap (effective joint space) formed between the bone and the stem and accelerate osteolysis of the bone, a serious drawback for patient recuperation.

Further, in the case of a stem with the entirety of its surface being porous, a substantial part of any vertical force is transferred to the lower side thereof, thereby stress-shielding the upper side of the femur, causing bone resorption from low stress. Furthermore, in the case where only the upper part of the stem is made porous, the lower part can produce a micro movement due to the weak connection, resulting in pain to the patient.

In the case of revision surgery due to a problem with a cementless artificial joint, removal of the stem is very challenging because the connection between the joint stem and the femur is a physiological one, based on the in-growth or on-growth of the bone into or onto the porous surface of the metal stem.

The present invention is aimed at reducing or eliminating problems associated with the techniques as described above. One object of the present invention is to provide, in combination, a jacket and stem for a cementless artificial joint, wherein shear forces, harmful to the service life of an artificial joint, can be markedly reduced and stress-shielding phenomena can also be markedly relieved. It is a further object of the invention to minimise osteolysis of a bone due to the infiltration of wear particles.

According to this invention there is provided a metal jacket and stem, in combination, as defined in claim 1.

According to said claim, there is provided in combination, a metal jacket and a stem for a cementless artificial joint, said jacket enclosing at least a part of the cementless artificial joint stem, said combination being adapted to be inserted longitudinally in an opening formed in a bone canal of a human body, the surface of said jacket having a surface-processed metal layer to thereby interlock the bone with the metal jacket as the bone grows onto the metal surface and the bottom part of the jacket presenting a space in which the stem is slidable, characterised in that the inside of said jacket is formed of wear resisting plastics enclosing the stem.

Preferably, in one embodiment, the metal layer is formed by wires arranged in a zigzag or meandering form.

Preferably, the metal layer is formed of a surface-roughened thin metal bag having a number of small openings on, say, the circumferential surface of the bag in, say, the longitudinal direction.

In another preferred embodiment of the invention, the metal layer comprises a thin corrugated metal bag.

The inner surface of the metal jacket is formed with a plastic film having a high resistance to abrasion.

Another aspect of the invention provides a cementless artificial joint (20) comprising a cementless artificial joint stem which is integrally formed of a head, neck and stem portion, and a metal jacket in accordance with the invention.

Where an artificial joint, equipped with an metal jacket for an artificial joint stem according to the invention is inserted into a long bone, a predominantly compressive force between the bone and the metal jacket suppresses the formation of fibrous tissue membrane, to activate or fortify the bone, and reduces the shear stress detrimental to the bone at the interface between the bone and the metal jacket and turn it into a beneficial compressive force. The compressive force acts to minimise the osteolysis of a bone, say the femur, which would otherwise occur due to the infiltration of wear particles by the formation of a gap between the bone and the metal jacket.

In an artificial joint equipped with a metal jacket for a cementless artificial joint stem according to the invention, the vertical force is mostly transferred into the bone hoop stress, which induces a tensile stress large enough to physiologically activate bone growth (Wolfe's law) even at the proximal region of the bone, alleviating the phenomena of stress shielding drastically.

The generation of wear particles from sliding contact with the stem can be minimised by placing a wear resistant plastics film, preferably polyethylene film, on the inner surface of the jacket.

Furthermore, the metal jacket for a cementless artificial joint according to the invention has the advantage that while securing a physiological connection with the bone after insertion, the wire can be easily separated from the plastics film, if present, at the time of revision surgery and unwound from the bone due to its inherent characteristics, minimising damage on or to the bone.

A metal jacket, of the invention, has the further advantage that direct contact between the bone and the stem is prevented by providing a strong and uniform tube-type layer. Breakage of the jacket due to, say, vertical motion of the stem, may be prevented by ensuring there is a space between the end of the metal jacket and the end of the stem.

The invention will now be described by way of example only and with reference to the accompanying drawings in which:
Figure 1 is a perspective view of an artificial joint according to the prior art discussed above;
Figure 2 is a perspective view of a metal jacket according to the invention;
Figure 3 is a cross section along the line A-A of the metal jacket of Figure 2;
Figure 4 is an artificial joint stem as inserted in the metal jacket of Figure 2, and
Figure 5 is the artificial joint of Figure 4 as inserted in the bone canal in a human body.

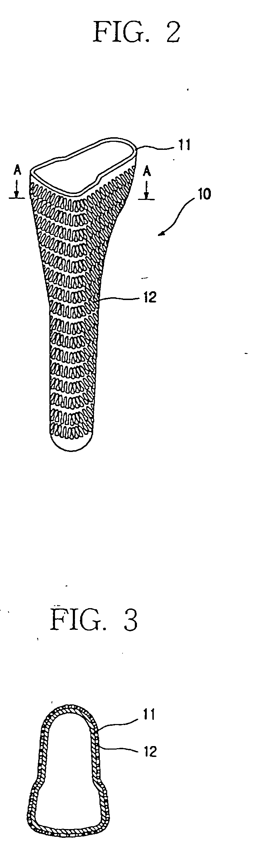

As can be seen in Figures 2 and 3, a metal jacket 10 for a cementless artificial joint stem, according to the invention, is shaped, dimensioned and arranged to enclose a stem when it is inserted therein. The metal jacket 10 is dimensioned so that the stem can slide down vertically, as shown, in its diameter, to match the outer diameter of the stem. The metal jacket 10 is a little longer than the stem to provide a predetermined space between the lower end of the stem and the lower end of the metal jacket 10. This space prevents the lower tip of the stem from touching the lower end of the metal jacket 10 when the stem is received therein. The space is to prevent damage to the metal jacket 10, if the stem sinks downward, once inserted.

The metal jacket 10 comprises a plastics layer 11, such as an ultrahigh molecular weight polyethylene or equivalent plastics layer to minimise abrasion caused by vertical sliding of the stem within the jacket 10. The surface of the plastics layer 11 is formed additionally with a surface-processed metal layer. The metal layer is formed of wires 12 of titanium or another suitable metal or alloy. Prior to construction of the jacket 10, the surfaces of the wires 12 are roughened by grit blasting, plasma spray or other processing. The wires 12 wrap the plastic layer 11 in a zigzag or meandering fashion. Wrapping metal wire 12 around the surface of the metal jacket 10 in the zigzag or meandering fashion is intended to translate the vertical force from the head of the joint to the compressive stress at the bone canal and to increase the surface area so that physiological interface interlocking can be secured with the on-growth or in-growth of the bone. Simultaneously, the hoop stress can be better transferred to the bone, such as the femur.

The metal layer 12 can be formed, instead of wires, by a surface-roughened thin metal bag having small openings on the surface thereof, or with a number of folds along the circumference in the longitudinal direction. The openings or folds allow radial expansion of the metal bag to improve transference of hoop stress to the femur.

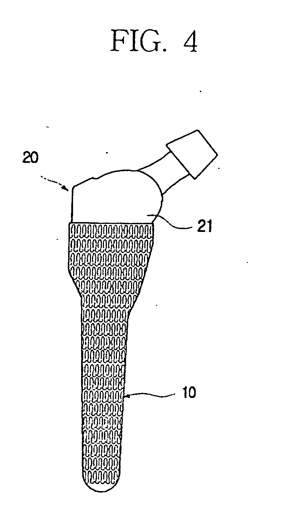

Now, the procedure of inserting an artificial joint by using the jacket 10 so constructed will be described. As can be seen from Figure 4, the stem 21 of a conventional artificial joint 20, the surface of which is polished, is first inserted into a metal jacket 10. Then, a prescribed space between the lower end of a stem 21 and the lower end of a metal jacket 10 is formed, to enable the stem 21 to be slidable relative to the metal jacket 10, as mentioned earlier.

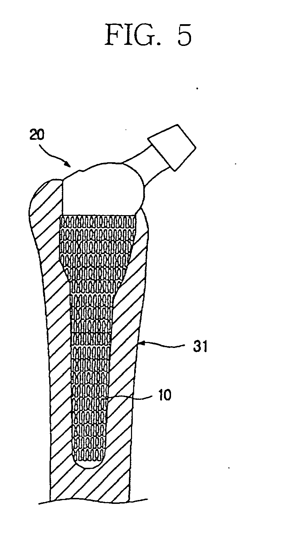

The artificial joint 20, comprising the metal jacket 10 placed over the stem 21, is ready to be inserted into a bone canal in a human body. To that end, the bone canal is reamed to form an opening in which the artificial joint 20 is inserted, and thus the stem 21 housed in the metal jacket 10 is inserted therein, as seen in Figure 5. Over time, the metal jacket 10 wound with metal wire 12 becomes physiologically interlocked with adjacent bone 31, as the bone 31 makes on-growth on the metal wire. Consequently, the bone 31 and the metal jacket 10 are strongly combined, while the stem 21 can slide down in relation to the metal jacket 10.

The metal jacket 10 according to the present invention can be applied to all kinds of artificial joint including the hip joint, knee joint, shoulder joint and the like.

## Claims

1. A metal jacket (10) and stem, in combination, for a cementless artificial joint, said jacket enclosing at least a part of said cementless artificial joint stem, said combination adapted to be inserted longitudinally in an opening formed in a bone canal of a human body, the surface of said jacket having a surface-processed metal layer (12) to thereby interlock the bone with the metal jacket as the bone grows onto the metal surface and the bottom part of the jacket (10) presenting a space in which the stem is slidable, **characterised in that** the inside of said jacket Is formed of wear resisting plastics enclosing the stem.

2. A metal jacket (10) and stem combination according to Claim 1, wherein the metal layer (12) is formed by metal wires wound in a zigzag or meandering form.

3. A metal jacket (10) and stem combination according to Claim 1, wherein the metal layer comprises a surface-processed thin metal bag with a plurality of small openings.

4. A metal jacket (10) and stem combination according to Claim 3, wherein the metal layer comprises a surface-processed thin metal bag with a plurality of folds formed along the circumference.

5. A cementless artificial joint (20) comprising:
a cementless artificial joint stem which is integrally formed of a head, neck and stem portion, and a metal jacket (10) according to any one of Claims 1 to 4.

## Patentansprüche

1. Kombination aus einem Metallmantel (10) und einem Schaft für ein zementloses künstliches Gelenk, wobei der Mantel wenigstens einen Teil des zementlosen künstlichen Gelenkschafts einschließt, wobei die Kombination so angepasst ist, dass sie in Längsrichtung in eine in einem Knochenkanal eines menschlichen Körpers ausgebildete Öffnung eingeführt werden kann, wobei die Oberfläche des Mantels eine oberflächenbehandelte Metallschicht (12) aufweist, um hierdurch den Knochen mit dem Metallmantel zu verzahnen, während der Knochen auf die metallene Oberfläche wächst und der Bodenteil des Mantels (10) eine Öffnung aufweist, in der der Schaft verschiebbar ist, **dadurch gekennzeichnet, dass** die Innenseite des Mantels aus verschleißbeständigem, den Schaft umschließendem Kunststoff gebildet ist.

2. Metallmantel- (10) und Schaftkombination nach Anspruch 1, wobei die Metallschicht (12) durch zickzackförmig oder schlangenförmig gewundene Metalldrähte gebildet ist.

3. Metallmantel- (10) und Schaftkombination nach Anspruch 1, wobei die Metallschicht eine oberflächenbehandelte dünne Metalltasche mit einer Vielzahl von kleinen Öffnungen umfasst.

4. Metallmantel- (10) und Schaftkombination nach Anspruch 3, wobei die Metallschicht eine oberflächenbehandelte dünne Metalltasche mit einer Vielzahl von entlang dem Umfang ausgebildeten Falten aufweist.

5. Zementloses künstliches Gelenk (20) mit den folgenden Bestandteilen:
einem zementlosen künstlichen Gelenkschaft, der einstückig aus einem Kopf-, einem Nacken- und einem Schaftteil gebildet ist, und einem Metallmantel (10) gemäß einem der Ansprüche 1 bis 4.

## Revendications

1. Gaine métallique (10) et tige, en combinaison, pour une articulation artificielle sans ciment, ladite gaine entourant au moins une partie de ladite tige d'articulation artificielle sans ciment, ladite combinaison étant adaptée pour être insérée longitudinalement dans une ouverture formée dans un canal osseux d'un corps humain, la surface de ladite gaine comportant une couche métallique (12) traitée en surface pour verrouiller de ce fait l'os avec la gaine métallique alors que l'os se développe sur la surface métallique et la partie inférieure de la gaine (10) présentant un espace dans lequel la tige peut coulisser, **caractérisées en ce que** l'intérieur de ladite gaine est formé de matières plastiques résistant à l'usure entourant la tige.

2. Combinaison de gaine métallique (10) et de tige selon la revendication 1, dans laquelle la couche métallique (12) est formée par des fils métalliques enroulés en zigzag ou en une forme sinueuse.

3. Combinaison de gaine métallique (10) et de tige selon la revendication 1, dans laquelle la couche métallique comprend un sac métallique mince traité en surface comportant une pluralité de petites ouvertures.

4. Combinaison de gaine métallique (10) et de tige selon la revendication 3, dans laquelle la couche métallique comprend un sac métallique mince traité en surface comportant une pluralité de plis formés le long de la circonférence.

5. Articulation artificielle sans ciment (20) comprenant :
une tige d'articulation artificielle sans ciment qui est formé d'un seul tenant de parties de tête, de col et de tige, et une gaine métallique (10) selon l'une quelconque des revendications 1 à 4.
